Europäisches Patentamt

European Patent Office

Office européen des brevets

(11) Veröffentlichungsnummer: **0 031 573**
**B1**

(12) **EUROPÄISCHE PATENTSCHRIFT**

(45) Veröffentlichungstag der Patentschrift:
08.06.83

(21) Anmeldenummer: **80108101.9**

(22) Anmeldetag: **22.12.80**

(51) Int. Cl.³: **C 07 C 46/04**, C 07 C 50/10

(54) Verfahren zur Herstellung von 2-Methylnaphthochinon-1,4.

(30) Priorität: **29.12.79 DE 2952709**

(43) Veröffentlichungstag der Anmeldung:
**08.07.81 Patentblatt 81/27**

(45) Bekanntmachung des Hinweises auf die Patenterteilung:
**08.06.83 Patentblatt 83/23**

(84) Benannte Vertragsstaaten:
**CH DE FR GB IT LI NL**

(56) Entgegenhaltungen:
**FR-A-1 558 481**

**,,Chemical Abstract'', Bd. 41 (1947), Spalte 2085, Zusammenfassung g-h, Columbus (Ohio), USA.**
**,,Chemical Abstracts'', Bd. 74 (1971), S. 482, Zusammenfassung 99716a, Columbus (Ohio), USA.**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Puetter, Hermann, Dr., Landauer Strasse 59, D-6730 Neustadt (DE)**
Erfinder: **Bewert, Wolfgang, Dr., Lorscher Ring 8C, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 2-Methylnaphthochinon-1,4

Diese Erfindung betrifft ein Verfahren zur Herstellung von 2-Metylnaphthochinon-1,4 von hoher Reinheit durch Chromsäureoxidation von 2-Methylnaphthalin.

Bekanntlich lässt sich 2-Methylnaphthochinon-1,4 durch Behandlung von 2-Methylnaphthalin mit Chromsäure in Essigsäure herstellen. Bei dem in der US-PS Nr. 2331725 beschriebenen Verfahren verfährt man z.B. so, dass man eine Lösung des 2-Methylnaphthalins in Essigsäure zu einer Lösung von Chromsäure in 80%iger Essigsäure gibt.

Da bei diesem Verfahren 2-Methylnaphthochinon-1,4 in unbefriedigender Ausbeute und Reinheit erhalten wird, wurde der Vorschlag gemacht, das 2-Methylnaphthalin in einem organischen Lösungsmittel der Chromsäureoxidation zu unterwerfen. Nach diesem in der US-PS Nr. 2402226 beschriebenen Verfahren, bei dem man als Lösungsmittel Tetrachlorkohlenstoff verwendet, sollen Ausbeuten von 85 bis 90% erzielt werden. Dies wird aber nur erreicht, wenn man Tetrachlorkohlenstoff in grossem Überschuss, wie der 60fachen Menge, bezogen auf 2-Methylnaphthalin, anwendet. Werden günstigere Gewichtsverhältnisse gewählt, so verringern sich die Ausbeuten erheblich.

Erschwerend kommt hinzu, dass die nach diesem Verfahren erhältlichen Rohprodukte nur auf umständliche Weise zu isolieren sind und in unreiner Form anfallen.

Man hat deshalb vorgeschlagen, die so gewonnenen Produkte gesondert zu reinigen, z.B. durch das in der US-PS Nr. 3079405 beschriebene mehrstufige Reinigungsverfahren, das den Einsatz weiterer Lösungsmittel verlangt.

Aus der in „Chemical Abstract", Bd 41 (1947), Spalte 2085 g-h, erwähnten SU-A Nr. 66489 ist ein Verfahren zur Herstellung von 2-Methylnaphthochinon-1,4 bekannt, bei dem man 2-Methylnaphthalin in schwefelsaurem Milieu bei 92 bis 95° C mit Dichromat oxidiert. Man verfährt dabei so, dass man das 2-Methylnaphthalin vorlegt, nach Zugabe von Wasser und Dichromat das Gemisch erhitzt, wobei das 2-Methylnaphthalin schmilzt, bei 85 bis 90° C die Schwefelsäure unter Rühren zugigt, weitere 4 h bei 92 bis 95° C rührt, das 2-Methylnaphthochinon-1,4 isoliert und aus Äthanol umkristallisiert. Er werden Ausbeuten von 35 bis 40% der Theorie erhalten.

Nach dem in der polnischen Patentschrift Nr. 61085 beschriebenen Verfahren, bei dem man 2-Methylnaphthalin in schwefelsaurer Lösung bei 70 bis 85° C mit Chromsäure behandelt, werden zwar bessere Ausbeuten (gegenüber der SU-A-66489) erhalten. Man muss aber auch hier umkristallisieren, um reines 2-Methylnaphthochinon-1,4 zu erhalten.

Es wurde nun gefunden, dass man bei der Herstellung von 2-Methylnaphthochinon-1,4 durch Behandlung von 2-Methylnaphthalin mit einer Lösung von Chromsäure oder eines Salzes der Chromsäure in einer wässerigen Mineralsäure das 2-Methylnaphthochinon in höherer Ausbeute und Reinheit erhält, wenn man die Behandlung bei 40 bis 70° C und in Abwesenheit eines organischen Lösungsmittels vornimmt, wobei man das 2-Methylnaphthalin im Reaktionsgemisch durch mechanische Nasszerkleinerung fein verteilt. Nach dem erfindungsgemässen Verfahren wird das 2-Methylnaphthochinon-1,4 überraschenderweise in so hoher Reinheit erhalten, dass sich eine besondere Reinigungsoperation erübrigt.

Das 2-Methylnaphthalin wird mit Chromsäure oder einem Salz der Chromsäure, wie Natriumdichromat oder Kaliumdichromat oxidiert. Dabei wird das Oxidationsmittel in wässeriger Mineralsäure, wie Phosphorsäure oder Schwefelsäure, angewendet. Die Säurekonzentration beträgt z.B. 5 bis 70, vorzugsweise 15 bis 60 Gew.%. Die Konzentration von Cr VI in der wässerigen Mineralsäure beträgt z.B. 0,1 bis 4, vorzugsweise 0,5 bis 2 mol/kg.

Die Chromsäure oder ihre Salze werden zweckmässig im Überschuss angewendet. Vorteilhaft verwendet man einen Überschuss an Cr VI von 100 bis 500%, vorzugsweise 150 bis 400% der Theorie. Zur Erzielung einer guten Raum-Zeit-Ausbeute hat es sich als zweckmässig erwiesen, wenn sich am Ende der Reaktion noch Restmengen von Cr VI im Reaktionsgemisch befinden, z.B. etwa 40 bis 120% der theoretisch benötigten Menge.

Man führt die Behandlung des 2-Methylnaphthalins mit dem Oxidationsmittel bei Temperaturen von 40 bis 70, insbesondere 45 bis 55° C durch. Die Reaktionstemperatur hält man zu Beginn der Reaktion durch Kühlen aufrecht. Im Verlauf der Reaktion kann die Temperatur durch die Zulaufgeschwindigkeit der mineralsauren Lösung des Oxidationsmittels gesteuert werden. Die Reaktionsdauer der einzelnen Anzätze liegt meist zwischen 2 und 10, vorzugsweise zwischen 3 und 6 h.

Nach dem erfindungsgemässen Verfahren wird das 2-Methylnaphthalin im Reaktionsgemisch durch mechanische Nasszerkleinerung fein verteilt. Man erreicht dies durch die Anwendung an sich bekannter Verfahren der mechanischen Nasszerkleinerung, wie Rühren, Umpumpen, Mahlen oder Ultraschallbehandlung. Vorteilhaft verwendet man Rührwerke, die Scheibenrührer oder Dissolver. Als Mahlhilfsmittel haben sich Seesand oder Glaskugeln bewährt.

Ein besonderer Vorteil des erfindungsgemässen Verfahrens ergibt sich aus der Möglichkeit, die Chromsalzlösung nach der Isolierung des Naphthachinons elektrochemisch zu regenerieren. Man kann danach die wässerige mineralsaure Cr-VI-Lösung unmittelbar für eine erneute Oxidation einsetzen. Dadurch lässt sich eine Handhabung fester Cr-VI-Salze (toxische Stäube) und eine Freisetzung von Chromsalzen an die Umwelt auf ein Minimum reduzieren.

Man isoliert das 2-Methylnapthochinon-1,4 aus dem Reaktionsgemisch, beispielsweise durch Absaugen oder durch Extraktion mit Lösungsmitteln, wie Toluol, Benzol, Hexan oder Dichlormethan. Das isolierte Produkt ist hellgelb, neigt nicht zum Zusammenbacken und hat einen Schmelzpunkt von ⩾104°C. Durch Gaschromatographie wird eine Reinheit von 99% ermittelt. Die Ausbeuten betragen 60 bis über 70%.

Es ist ausserordentlich überraschend, dass nach dem erfindungsgemässen Verfahren das ohne den Einsatz eines organischen Lösungsmittels durchgeführt wird, 2-Methylnaphthochinon-1,4 in so hoher Ausbeute und Reinheit erhalten wird.

*Beispiel 1*

In einem 2-l-Vierhalskolben, der mit 4 seitlichen Einbuchtungen als Wellenbrecher, Tropftrichter, Innenthermometer und einem Rührwerk ausgerüstet ist, werden 37,4 g (0,37 mol) $CrO_3$ in 250 ml 20%iger Schwefelsäure und 50 g Seesand mit 26,0 g (0,18 mol) 98%igem 2-Methylnaphthalin unter heftigem Rühren (1500 tr/min) auf 50°C erhitzt. Nach abklingen der Reaktionswärme gibt man weitere 112 g $CrO_3$ in 750 ml 20%iger Schwefelsäure allmählich hinzu, wobei man die Temperatur auf 50°C hält. Nach 5 h ist die Reaktion beendet. Das Produkt wird zusammen mit dem Seesand abgesaugt. Anschliessend wird es in Aceton aufgenommen. Die Acetonphase wird getrocknet und im Rotationsverdampfer eingedampft. Ausbeute: 22,2 g 2-Methylnaphthochinon-1,4 in Form hellgelber Kristalle (71%), Fp. 104 bis 105°C, Reinheit nach GC: >99,5%.

*Beispiel 2*

Man verfährt wie im Beispiel 1 beschrieben, wobei man jedoch anstelle von Seesand 100 g Glaskugeln (∅ 3 mm) verwendet. Während der Reaktion werden mehrfach Proben zur Titration von Cr VI und zur GC-Bestimmung entnommen, wofür, insgesamt 5% des Ansatzes verbraucht werden. Nach der GC-Analyse ist die Reaktion nach 3 h beendet. Ausbeute: 19,0 g 2-Methylnaphthochinon-1,4 in Form hellgelber Kristalle (63%, bezogen auf Gesamtansatz: 66%). Fp. 105 bis 106°C. Durch Extraktion der Mutterlauge mit Methylenchlorid werden weitere 0,65 g 2-Methylnaphthochinon-1,4 isoliert (2% d.Th.).

*Beispiel 3*

Man verfährt wie im Beispiel 1, wobei man eine Chromsäurelösung verwendet, die aus einer Serie von insgesamt 46 Ansätzen gemäss Beispiel 1 stammte und die nach jedem dieser Ansätze elektrochemisch regeneriert wurde. Man erhält 2-Methylnaphthochinon-1,4 in einer Ausbeute von 63% und einer Reinheit (GC) von 99% mit dem Schmelzpunkt ~104°C. Umgesetzte Chromsäuremenge: 0,99 mol (Δ 270% d.Th.). Die Chromsäurelösung wurde an einer $PbO_2$/Pb-Anode in einer geteilten Zelle bei einer Stromdichte von 5 A/dm², einer Stromausbeute von 57% und einem Energiebedarf von 5,45 kWh/kg $CrO_3$ anodisch regeneriert.

*Beispiel 4*

Aus 95,6 g $CrO_3$, 345,5 g konz. Schwefelsäure und 771,5 ml Wasser wird eine Chromsäurelösung hergestellt. 200 ml dieser Lösung werden in einem Reaktionskolben vorgelegt, der mit einem Ultra-Turrax-Rührer ausgerüstet ist. Man gibt 35,5 g geschmolzenes 2-Methylnaphthalin hinzu, setzt den Rührer in Betrieb und erwärmt das Gemisch auf 65 bis 70°C. Die Reaktionsdauer beträgt 5 h, wobei man nach jeweils 1 h weitere 200 ml der Chromsäure und zuletzt (nach insgesamt 4 h) den von der Chromsäurelösung verbliebenen Rest hinzugibt. Das Reaktionsgemisch wird bei einer Temperatur von 50 bis 60°C mit 300 ml Totuol extrahiert. Die Toluolphase wird mit Natriumsulfat getrocknet und zur Trockene eingeengt. Es werden 28,1 g (65%) 2-Methylnaphthochinon-1,4 mit einer Reinheit von 99% erhalten.

*Beispiel 5*

In einem 10-l-Glasgefäss, das mit Heizmantel, einem Scheibenrührer und drei Strömungsunterbrechern ausgerüstet ist, wird das schwefelsaure Filtrat, das nach Beispiel 1 erhalten wird, vorgelegt.

Bei 47°C werden dann unter gutem Rühren 75,2 g 98%iges 2-Methylnaphthalin in der Lösung suspendiert. Anschliessend gibt man innerhalb von 1,5 h bei 50°C ±1°C und einer Rührgeschwindigkeit von 470 tr/min eine Lösung von 299 g $CrO_3$ in 2 l 35%iger Schwefelsäure zu. Man hält das Reaktionsgemisch noch, 3,5 h bei konstanter Rührgeschwindigkeit und Temperatur. Dann wird abgekühlt und abgelaugt. Der gelbe Niederschlag wird mit Wasser gewaschen und getrocknet.

Man erhält 51,0 g 2-Methylnaphthochinon-1,4 (Ausbeute 57%) in einer Reinheit von 99%. Durch Spülen des Reaktionsgefässes mit Methylenchlorid und Trocknen und Eindampfen der Methylenchloridphase können restliche Anteile an 2-Methylnaphthochinon gewonnen werden, so dass eine Gesamtausbeute von 65% erreicht wird.

**Patentansprüche**

1. Verfahren zur Herstellung von 2-Methylnaphthochinon-1,4 von hoher Reinheit durch Behandlung von 2-Methylnaphthalin mit einer Lösung von Chromsäure oder eines Salzes der Chromsäure in einer wässerigen Mineralsäure, dadurch gekennzeichnet, dass man die Behandlung bei 40 bis 70°C und in Abwesenheit eines organischen Lösungsmittels vornimmt, wobei man das 2-Methylnaphthalin im Reaktionsgemisch durch mechanische Nasszerkleinerung fein verteilt.

2. Verfahren nach Anspruch 1, dadurch gekennzeichnet, dass man die Behandlung bei 45 bis 55°C vornimmt.

## Claims

1. A process for the preparation of very pure 2-methyl-1,4-naphthoquinone by treating 2-methylnaphthalene with a solution of chromic acid or of a chromic acid salt in an aquenous mineral acid, wherein the treatment is carried out at 40 to 70° C in the absence of an organic solvent, the 2-methylnaphthalene being finely dispersed in the reaction mixture by mechanical wet comminution.

2. A process as claimed in claim 1, wherein the treatment is carried out at 45 to 55° C.

## Revendications

1. Procédé de préparation de 2-méthyl-naphtoquinone-1,4 à haute pureté par traitement du 2-méthylnaphtalène à l'aide d'une solution d'acide chromique ou d'un sel de l'acide chromique dans un acide minéral aqueux, caractérisé en ce que l'on procède au traitement à une température de 40 à 70° C et en l'absence de solvant organique, en répartissant finement le 2-méthylnaphtalène dans le mélange de réaction par un broyage mécanique au mouillé.

2. Procédé selon la revendication 1, caractérisé en ce que l'on procède au traitement à une température de 45 à 55° C.